(19) Europäisches Patentamt
European Patent Office
Office européen des brevets

(11) Veröffentlichungsnummer: 0 430 881 A2

(12) EUROPÄISCHE PATENTANMELDUNG

(21) Anmeldenummer: 90810897.0

(22) Anmeldetag: 20.11.90

(51) Int. Cl.[5]: C07D 311/86, C07D 335/16, C07D 219/06, C07D 403/12, C07D 405/12, C09K 9/00, G03F 7/00

(30) Priorität: 29.11.89 CH 4270/89

(43) Veröffentlichungstag der Anmeldung: 05.06.91 Patentblatt 91/23

(84) Benannte Vertragsstaaten: BE CH DE FR GB IT LI NL SE

(71) Anmelder: CIBA-GEIGY AG
Klybeckstrasse 141
CH-4002 Basel (CH)

(72) Erfinder: Fischer, Evelyn, Dr.
Schutzackerstrasse 64
W-7858 Weil am Rhein (DE)
Erfinder: Fischer, Walter, Dr.
Vogesenstrasse 77
CH-4153 Reinach (CH)
Erfinder: Finter, Jürgen, Dr.
Zasiusstrasse 100
W-7800 Freiburg (DE)
Erfinder: Meier, Kurt, Dr.
Stefanstrasse 66
CH-4106 Therwil (CH)
Erfinder: Roth, Martin, Dr.
Oberdorf
CH-1735 Giffers (CH)

(54) **Photochrome Verbindungen, Verfahren zu deren Herstellung und deren Verwendung.**

(57) Verbindungen der Formel I

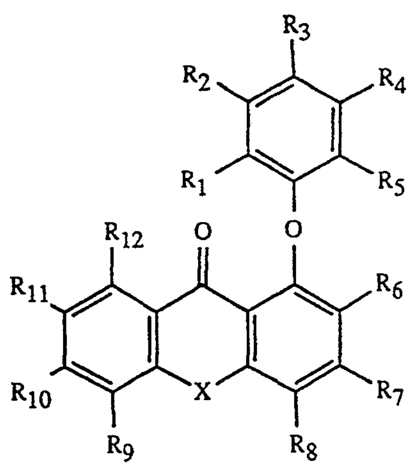

(I),

worin
X für O, S, SO, $SO_2$ oder $NR_{13}$ steht, $R_{13}$ z.B. Methyl bedeutet, $R_1$ bis $R_{12}$ unabhängig voneinander z.B. H, $C_1$-$C_{12}$-Alkyl, $C_1$-$C_{12}$-Alkoxy, Halogen oder —CN darstellen. Die Verbindungen sind lichtempfindlich und photochrom und eignen sich als Photosensibilisatoren und gleichzeitig Farbindikatoren, sowie als photoschaltbare Farbfilter.

EP 0 430 881 A2

# PHOTOCHROME VERBINDUNGEN, VERFAHREN ZU DEREN HERSTELLUNG UND DEREN VERWENDUNG

Die vorliegende Erfindung betrifft photochrome, in 1-Stellung mit gegebenenfalls substituiertem Phenoxy substituierte Acridone, Xanthone, Thioxanthone, Thioxanthonsulfoxide und Thioxanthonsulfodioxide, ein Verfahren zu deren Herstellung, eine photoempfindliche Zusammensetzung, und die Verwendung dieser photochromen Verbindungen als Photosensibilisatoren und Farbindikatoren oder als photoschaltbare Farbfilter.

Es ist bekannt, dass Thioxanthone (siehe US-A-4 506 083) oder Thioxanthonsulfoxide bzw. Thioxanthonsulfodioxide (siehe JP Sho 58-120605) Photoinitiatoren oder Photosensibilisatoren für strahlungsempfindliche polymerisierbare Systeme sind. B.K. Manukian beschreibt im Helv. Chim. Acta. Vol. 59, Fasc. 7 (1976), S. 2609 bis 2614 in 1-Stellung mit gegebenenfalls substituiertem Phenoxy substituierte 4-Nitro-acridone als Farbstoffe mit hoher Lichtechtheit. In der DE-A-919 107 ist 1-Phenoxy-4-methyl-xanthen-9-on als pharmazeutischer Wirkstoff beschrieben.

Ein Gegenstand der Erfindung sind Verbindungen der Formel I

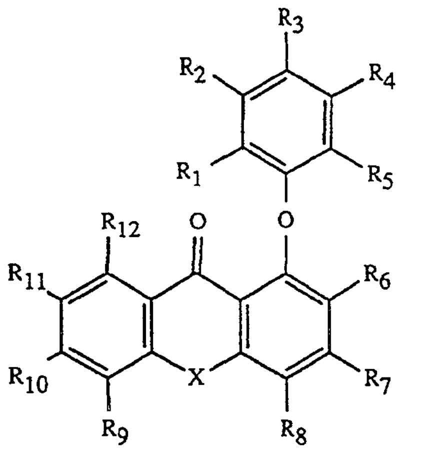

(I),

worin

X für O, S, SO, $SO_2$ oder $NR_{13}$ steht,

$R_1$ bis $R_5$ unabhängig voneinander H, $C_1$-$C_{12}$-Alkyl, $C_1$-$C_{12}$-Alkoxy, $C_1$-$C_{12}$-Alkylthio, $C_1$-$C_4$-Alkyl-SO—, $C_1$-$C_4$-Alkyl-$SO_2$—, Halogen, $CF_3$, —CN, —$NO_2$, —OH, —$COOR_{14}$, —$CON(R_{15})_2$, oder —$N(R_{15})_2$ oder den Rest der Formel

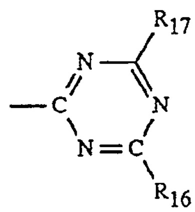

bedeuten, worin

$R_{16}$ und $R_{17}$ unabhängig voneinander $C_1$-$C_6$-Alkyl, Phenyl, $C_1$-$C_6$-Alkylphenyl oder ($C_1$-$C_6$-Alkyl)$_2$-phenyl bedeuten, oder $R_3$ und $R_4$ oder $R_4$ und $R_5$ je zusammen —CH=CH-CH=CH— darstellen,

$R_6$ bis $R_{12}$ unabhängig voneinander H, $C_1$-$C_{12}$-Alkyl, $C_1$-$C_{12}$-Alkoxy, $C_1$-$C_{12}$-Alkylthio, $C_1$-$C_{12}$-Alkyl-SO—, $C_1$-$C_{12}$-Alkyl-$SO_2$—, in 1-Stellung mit insgesamt ein oder zwei —CN und/oder —$COOR_{14}$ substituiertes $C_1$-$C_4$-Alk-1-yl oder Benzyl, $C_6$-$C_{10}$-Aryl, $C_7$-$C_{14}$-Aralkyl, $C_6$-$C_{10}$-Aryl-CO, $C_6$-$C_{10}$-Aryloxy, $C_6$-$C_{10}$-Arylthio, $C_6$-$C_{10}$-Aryl-SO—, $C_6$-$C_{10}$-Aryl-$SO_2$, $C_7$-$C_{14}$-Aralkyloxy, $C_7$-$C_{14}$-Aralkylthio,, $C_7$-$C_{14}$-Aralkyl-SO—, $C_7$-$C_{14}$-Aralkyl-$SO_2$—, $C_1$-$C_{18}$-Acyl-O—, —$COOR_{14}$, —$CON(R_{15})_2$, $C_1$-$C_{18}$-Acyl-$NR_{15}$—, $(R_{15})_2N$—, Halogen, —$CF_3$ oder —CN oder je zwei benachbarte Reste von $R_6$ bis $R_{12}$ die Gruppen —CO-O-CO— oder —CO-$NR_{13}$-CO— bedeuten, wobei die Arylreste und Arylgruppen enthaltenden Reste unsubstituiert oder mit Halogen, —CN, —$CF_3$, $C_1$-$C_6$-Alkylthio, $C_1$-$C_6$-Alkoxy, $C_1$-$C_6$-Alkyl, —$COOR_{14}$, —CO-$N(R_{15})_2$ oder $C_1$-$C_{12}$-Acyl-O— substituiert sind,

$R_{13}$ H, $C_1$-$C_{12}$-Alkyl oder $C_1$-$C_{12}$-Acyl, Phenyl oder Benzyl darstellen, die unsubstituiert oder mit $C_1$-$C_6$-Alkyl, $C_1$-$C_6$-Alkoxy, $C_1$-$C_6$-Alkylthio, Halogen oder —$COOR_{14}$ substituiert sind,

beide $R_{15}$ zusammen $C_4$-$C_8$-Alkylen, 3-Oxa-1,5-pentylen, 3-Thia-1,5-pentylen, 1,3-Butadien-1,4-diyl oder 2-Aza-1,3-Butadien-1,4-diyl bedeuten, oder die $R_{15}$ unabhängig voneinander H, $C_1$-$C_{12}$-Alkyl, oder unsubstituiertes oder mit $C_1$-$C_6$-Alkyl, $C_1$-$C_6$-Alkoxy, $C_1$-$C_6$-Alkylthio, Halogen oder —$COOR_{14}$ substituiertes Phenyl oder Benzyl darstellen, und $R_{14}$ H oder der um die OH-Gruppe verminderte Rest eines aromatischen oder aliphatischen Alkohols ist,
mit Ausnahme von 1-Phenoxy-4-methylxanthen-9-on.

$R_1$ bis $R_5$ in der Bedeutung von Alkyl, Alkoxy oder Alkylthio können linear oder verzweigt sein und enthalten bevorzugt 1 bis 6, besonders 1 bis 4 C-Atome. Beispiele sind Methyl, Ethyl und die Isomeren von Propyl, Butyl, Pentyl, Hexyl, Heptyl, Octyl, Nonyl, Decyl, Undecyl, Dodecyl, entsprechende Alkoxyreste und Alkylthioreste. Bevorzugt sind Methyl, Ethyl, n— oder i-Propyl, n—, i— oder t-Butyl, Methyloxy, Ethyloxy, n—, i-Propyloxy, n—, i— und t-Butyloxy, Methylthio und Ethylthio.

$R_1$ bis $R_5$ in der Bedeutung von $C_1$-$C_4$-Alkyl-SO-oder $C_1$-$C_4$-Alkyl-$SO_2$— können z.B. Methyl—, Ethyl—, n-Propyl— oder n-Butylsulfinyl oder —sulfonyl sein.

$R_1$ bis $R_5$ in der Bedeutung von Halogen können z.B. —F, —Cl, —Br oder —I sein.

$R_1$ bis $R_5$ in der Bedeutung von —$COOR_{14}$ sind vorzugsweise —COO-$C_1$-$C_{18}$-Alkyl ; von —$N(R_{15})_2$ sind bevorzugt —N($C_1$-$C_6$-Alkyl)$_2$ ; und von —$CON(R_{15})_2$ sind bevorzugt —CO-N($C_1$-$C_6$-Alkyl)$_2$ oder —CO-NH($C_1$-$C_6$-Alkyl).

$R_{16}$ und $R_{17}$ stehen bevorzugt für Methyl, Ethyl, Phenyl, Methylphenyl oder Dimethylphenyl.

In einer bevorzugten Ausführungsform stellen $R_1$ bis $R_5$ unabhängig voneinander H, $C_1$-$C_6$-Alkyl, $C_1$-$C_6$-Alkoxy, Halogen, —$CF_3$, —CN, —$NO_2$, —$COOR_{14}$, —OH, —$N(R_{15})_2$ oder $R_3$ und $R_4$ oder $R_4$ und $R_5$ je zusammen —CH=CH-CH=CH— dar, wobei $R_{14}$ lineares oder verzweigtes $C_1$-$C_{18}$-Alkyl und $R_{15}$ je $C_1$-$C_6$-Alkyl darstellen.

In einer anderen bevorzugten Ausführungsform stehen mindestens 2, besonders mindestens 3 der Reste $R_1$ bis $R_5$ für H.

$R_6$ bis $R_{12}$ in der Bedeutung von Alkyl, Alkoxy, Alkylthio, Alkyl-SO— oder Alkyl-$SO_2$— enthalten bevorzugt 1 bis 6, besonders 1 bis 4 C-Atome. Beispiele und weitere Bevorzugungen sind zuvor für $R_1$ bis $R_5$ angegeben.

$R_6$ bis $R_{12}$ in der Bedeutung von in 1-Stellung mit insgesamt ein oder zwei —CN und/oder —$COOR_{14}$ substituiertes $C_1$-$C_4$-Alk-1-yl oder Benzyl können z.B. Cyanomethyl, Dicyanomethyl, α-Cyanobenzyl, 1-Cyanoeth-1-yl, 1,1-Dicyanoeth-1-yl, (Methoxycarbonyl)-methyl, (Ethoxycarbonyl)methyl, Di(methoxycarbonyl)methyl, α-(Methoxycarbonyl)-benzyl, 1,1-Di(methoxycarbonyl)-eth-1-yl oder Cyano-(methoxycarbonyl)-methyl sein.

$R_6$ bis $R_{12}$ als Aryl sind bevorzugt Naphthyl und besonders Phenyl. $R_6$ bis $R_{12}$ in der Bedeutung von Arylgruppen enthaltenden Resten enthalten bevorzugt Naphthyl— und besonders Phenylgruppen.

$R_6$ bis $R_{12}$ in der Bedeutung von Aralkyl sind bevorzugt Phenyl-($C_1$-$C_4$-Alkyl), besonders Benzyl oder 1-Phenyleth-1-yl— oder 2-yl.

$R_6$ bis $R_{12}$ in der Bedeutung von Aryl-CO— sind bevorzugt Naphthyl-CO— und besonders Phenyl-CO—.

$R_6$ bis $R_{12}$ in der Bedeutung von Aryloxy sind bevorzugt Naphthyloxy und besonders Phenyloxy.

$R_6$ bis $R_{12}$ in der Bedeutung von Arylthio sind besonders Naphthylthio oder Phenylthio.

$R_6$ bis $R_{12}$ als Aryl-SO— oder Aryl-$SO_2$ sind bevorzugt Phenyl-SO— oder Phenyl-$SO_2$—.

$R_6$ bis $R_{12}$ als Aralkyloxy sind bevorzugt Phenyl($C_1$-$C_4$-alkyl)-O—, besonders Benzyloxy.

$R_6$ bis $R_{12}$ in der Bedeutung von Aralkylthio sind bevorzugt Phenyl($C_1$-$C_4$-alkyl)-S—, besonders Benzylthio.

$R_6$ bis $R_{12}$ in der Bedeutung von Aralkyl-SO— sind bevorzugt Phenyl($C_1$-$C_4$-alkyl)-SO—, besonders Benzyl-SO—.

$R_6$ bis $R_{12}$ in der Bedeutung von Aralkyl-$SO_2$— sind bevorzugt Phenyl($C_1$-$C_4$-Alkyl)-$SO_2$—, besonders Benzyl-$SO_2$—.

$R_6$ bis $R_{12}$ in der Bedeutung von $C_1$-$C_{18}$-Acyl-O— entsprechen bevorzugt der Formel $R_{19}$-CO-O, worin $R_{19}$ H oder unsubstituiertes oder mit Halogen, bevorzugt —F oder —Cl, —OH, $C_1$-$C_6$-Alkoxy oder $C_1$-$C_6$-Alkylthio substituiertes $C_1$-$C_{17}$-Alkyl, $C_2$-$C_{17}$-Alkenyl, $C_3$-$C_8$-Cycloalkyl, $C_6$-$C_{10}$-Aryl oder $C_7$-$C_{14}$-Aralkyl darstellen. Bevorzugt ist $R_{19}$ $C_1$-$C_{12}$-Alkyl, $C_2$-$C_{12}$-Alkenyl, Cyclopentyl, Cyclohexyl, Phenyl und Benzyl.

Bedeuten $R_6$ bis $R_{12}$ die Gruppe —$COOR_{14}$, so stellt $R_{14}$ besonders $C_1$-$C_{18}$-Alkyl dar.

Bedeuten $R_6$ bis $R_{12}$ die Gruppe —$CON(R_{15})_2$ oder die Gruppe —$CONHR_{15}$, so stellen die $R_{15}$ besonders $C_1$-$C_6$-Alkyl dar.

Bedeuten $R_6$ bis $R_{12}$ die Gruppe $C_1$-$C_{18}$-Acyl-$NR_{15}$—, so entspricht die Gruppe bevorzugt der Formel $R_{19}$-CO-$NR_{15}$—, worin $R_{19}$ die für die Gruppe $R_{19}$-CO-O— angegebenen Bedeutungen einschliesslich der Bevorzugungen hat, und $R_{15}$ bevorzugt H oder $C_1$-$C_6$-Alkyl bedeutet.

Bedeuten $R_6$ bis $R_{12}$ die Gruppe —$N(R_{15})_2$, so bedeuten die $R_{15}$ bevorzugt je $C_1$-$C_6$-Alkyl, Cyclohexyl, Phenyl, Benzyl, Tetra— oder Pentamethylen, 3-Oxa-1,5-Pentylen oder —CH=CH-CH=CH—.

Bei $R_6$ bis $R_{12}$ in der Bedeutung von Halogen handelt es sich bevorzugt um —F, —Cl oder —Br.

Bedeuten $R_6$ bis $R_{12}$ Arylgruppen oder Arylgruppen enthaltende Reste, so können diese unsubstituiert oder

ein- oder mehrfach, bevorzugt ein- oder zweifach, mit Halogen, bevorzugt —F, —Cl oder —Br, —CN, —OH, —$CF_3$, $C_1$-$C_6$-Alkylthio (z.B. Methyl— oder Ethylthio), $C_1$-$C_6$-Alkoxy (z.B. Methyloxy, Ethyloxy, n— oder i-Propyloxy, n—, i— oder t-Butyloxy), $C_1$-$C_6$-Alkyl (z.B. Methyl, Ethyl, n— oder i-Propyl, n—, i— oder t-Butyl), —CO-$OR_{14}$, worin $R_{14}$ bevorzugt H oder $C_1$-$C_{12}$-Alkyl ist, —$CON(R_{15})_2$, worin $R_{15}$ bevorzugt H oder $C_1$-$C_6$-Alkyl ist, oder $C_1$-$C_{12}$-Acyl-O—, bevorzugt $C_1$-$C_{11}$-Alkyl-CO-O—, substituiert sein.

$R_{13}$ in der Bedeutung von Alkyl enthält bevorzugt 1 bis 6, besonders 1 bis 4 C-Atome und ist z.B. Methyl oder Ethyl. $R_{13}$ in der Bedeutung von Acyl ist bevorzugt $R_{19}$-CO—, worin $R_{19}$ die zuvor angegebenen Bedeutungen hat und besonders $C_1$-$C_8$-Alkyl,Cyclohexyl, Phenyl oder Benzyl ist.

$R_{14}$ als Rest eines Alkohols enthält bevorzugt 1-30, besonders 1-20 und insbesondere 1-10 C-Atome. Bei $R_{14}$ als Rest eines aromatischen Alkohols kann es sich um $C_6$-$C_{10}$-Aryl, besonders Phenyl oder Phenyl-($C_1$-$C_4$-Alkyl), besonders Benzyl oder 1-Phenyleth-2-yl handeln, die mit 1 oder 2 $C_1$-$C_{18}$, besonders $C_1$-$C_{12}$-Alkylgruppen substituiert sein können.

Bei $R_{14}$ als aliphatischer Rest kann es sich um offenkettige oder cyclische Reste handeln, z.B. um $C_1$-$C_{20}$—, besonders $C_1$-$C_{18}$— und insbesondere $C_1$-$C_{12}$-Alkyl ; $C_3$-$C_8$-Cycloalkyl oder $C_3$-$C_8$-Cycloalkylmethyl, besonders Cyclopentyl, Cyclohexyl und Cyclohexylmethyl.

$R_{14}$ kann auch einen Polyoxaalkylenrest der Formel $\left(C_nH_{2n}\text{-}O\right)_m R_{18}$ bedeuten, worin n für eine ganze Zahl von 2 bis 6, besonders 2 bis 4 und insbesondere 2 oder 3 und m für eine Zahl von 1 bis 20, besonders 1 bis 12 und insbesondere 1 bis 6 stehen und $R_{18}$ H, $C_1$-$C_{18}$—, besonders $C_1$-$C_{12}$— und insbesondere $C_1$-$C_6$-Alkyl, $C_3$-$C_8$—, besonders $C_5$— oder $C_6$-Cycloalkyl oder —Cycloalkylmethyl ; oder Phenyl oder Benzyl darstellen, die mit 1 oder 2 Alkylgruppen mit 1 bis 12, besonders 1 bis 6 C-Atomen substituiert sein können.

$R_{15}$ in der Bedeutung von Alkyl enthält bevorzugt 1 bis 5, besonders 1 bis 4 C-Atome und ist insbesondere Methyl oder Ethyl. $R_{15}$ in der Bedeutung von substituiertem Phenyl oder Benzyl enthalten bevorzugt einen oder zwei Substituenten. Halogen und —$COOR_{14}$ sind als Substituenten bevorzugt —F, —Cl, —Br, —COOH und —COO($C_1$-$C_{12}$-Alkyl). Beide $R_{15}$ zusammen bedeuten als Alkylen bevorzugt Tetra- oder Pentamethylen.

In einer bevorzugten Ausführungsform stehen mindestens 2, besonders mindestens 3 und insbesondere mindestens 4 der Reste $R_6$ bis $R_{12}$ für H.

Eine bevorzugte Untergruppe von Verbindungen der Formel I sind solche, worin $R_6$ bis $R_{12}$ unabhängig voneinander H, $C_1$-$C_6$-Alkyl, $C_1$-$C_6$-Alkoxy, $C_1$-$C_6$-Alkylthio, $C_1$-$C_6$-Alkyl-SO—, $C_1$-$C_6$-Alkyl-$SO_2$—, in 1-Stellung mit insgesamt ein oder 2 —CN und/oder —$COOR_{14}$ substituiertes $C_1$— oder $C_2$-Alkyl, Phenyl, Phenyl-$C_1$-$C_4$-Alkyl, Phenyl-CO—, Phenyloxy, Phenylthio, Phenyl-SO—, Phenyl-$SO_2$—, Benzyloxy, Benzylthio, Benzyl-SO—, Benzyl-$SO_2$—, $C_1$-$C_8$-Acyl-$NR_{15}$—, —$COOR_{14}$, —$CON(R_{15})_2$, $(R_{15})_2N$—, —F, —Cl, —Br, —$CF_3$ oder —CN oder je zwei benachbarte Reste von $R_6$ bis $R_{12}$ zusammen —CO-O-CO— oder —CO-$NR_{13}$-CO— bedeuten,

$R_{13}$ $C_1$-$C_6$-Alkyl bedeuten, oder Phenyl oder Benzyl darstellen, die unsubstituiert oder mit $C_1$-$C_4$-Alkyl, $C_1$-$C_4$-Alkoxy, F, Cl, Br oder —$COOR_{14}$ substituiert sind,

$R_{14}$ H, lineares oder verzweigtes $C_1$-$C_{18}$-Alkyl, Phenyl, Benzyl oder

$\left(C_nH_{2n}\text{-}O\right)_m R_{18}$ ist, wobei n eine ganze Zahl von 2 bis 6 und m eine Zahl von 1 bis 20 darstellen, und $R_{18}$ H, $C_1$-$C_{18}$-Alkyl, Cyclohexyl, Phenyl oder Benzyl bedeutet, und $R_{15}$ $C_1$-$C_6$-Alkyl bedeutet, oder Phenyl oder Benzyl ist, die unsubstituiert oder mit $C_1$-$C_4$-Alkyl, $C_1$-$C_4$-Alkoxy, —F, —Cl, —Br oder —$COOR_{14}$ substituiert sind, oder beide $R_{15}$ zusammen 1,4-Butylen, 1,5-Pentylen, 3-Oxa-1,5-pentylen oder 1,3-Butadien-1,4-diyl darstellen.

Bevorzugte Gruppen von Verbindungen sind auch, worin in Formel I $R_6$ für H, —Cl, —Br oder Methyl steht, oder worin in Formel I $R_9$ H oder —Cl bedeutet, oder worin in Formel I $R_{11}$ H oder —Cl bedeutet, oder worin in Formel I $R_{10}$ und/oder $R_{12}$ —Cl, —Br, unsubstituiertes oder substituiertes Phenyloxy oder —COO-($C_1$-$C_{12}$-Alkyl) bedeuten, oder worin in Formel I $R_7$ und/oder $R_8$ für H, —F, —Cl, —$CF_3$, —Br, $C_1$-$C_4$-Alkyl, $C_1$-$C_4$-Alkoxy, Phenyl-CO—, α-Cyanobenzyl, $C_1$-$C_8$-Acyl-$NR_{15}$—, Pyrryl, —COO-($C_1$-$C_{12}$-Alkyl) stehen, oder Phenoxy, Phenylthio oder Phenylsulfonyl bedeuten, die unsubstituiert oder mit —F, —Cl, $C_1$-$C_4$-Alkyl, $C_1$-$C_4$-Alkoxy, —COOH oder —COO-($C_1$-$C_{12}$-Alkyl) substituiert sind, oder $R_7$ und $R_8$ zusammen

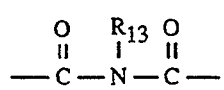

darstellen, und $R_{13}$ H oder $C_1$-$C_6$-Alkyl bedeutet, oder worin in Formel I $R_{13}$ $C_1$-$C_4$-Alkyl oder $C_1$-$C_8$-Acyl darstellt, oder Phenyl oder Benzyl ist, die unsubstituiert oder mit F oder —$COOR_{14}$ substituiert sind, und $R_{14}$ für H oder $C_1$-$C_{12}$-Alkyl steht, oder worin in Formel I $R_{14}$ H, $C_1$-$C_{18}$-Alkyl, $\left(C_nH_{2n}\text{-}O\right)_m H$, Phenyl, $C_1$-$C_8$-Alkylphenyl, Ben-

zyl, $C_1$-$C_6$-Alkylbenzyl oder Cyclohexyl bedeutet, n eine ganze Zahl von 1 bis 6 und m eine Zahl von 1 bis 20 darstellen, oder worin in Formel I $R_{15}$ H, $C_1$-$C_6$-Alkyl, Phenyl, Benzyl, oder beide $R_{15}$ zusammen Tetramethylen, Pentamethylen, 3-Oxa-1,5-pentylen oder 1,3-Butadien-1,4-diyl bedeuten.

Besonders bevorzugt sind Verbindungen der Formel I, worin $R_1$ bis $R_5$ H oder $R_1$, $R_2$, $R_4$ und $R_5$ H und $R_3$ —$CO_2C_2H_5$ bedeuten, $R_6$ und $R_8$ Cl und $R_7$ und $R_9$-$R_{12}$ H darstellen, und X für $NCH_3$ oder N-n-$C_3H_7$ steht.

Die erfindungsgemässen Verbindungen sind in an sich bekannter Weise durch nucleophile Substitution von 1-Nitro— oder 1-Halogenxanthonen, —Thioxanthonen, —Thioxanthonsulfoxiden, —Thioxanthonsulfodioxiden oder —Acridonen mit Phenolsalzen erhältlich. Ein weiterer Gegenstand der Erfindung ist ein Verfahren zur Herstellung von Verbindungen der Formel I, dadurch gekennzeichnet, dass man eine Verbindung der Formel II

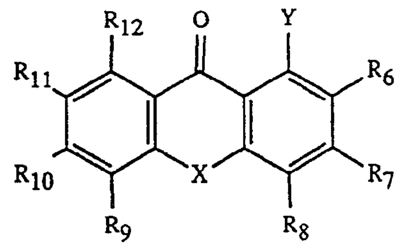

(II),

worin X und $R_6$ bis $R_{12}$ die in Anspruch 1 angegebenen Bedeutungen haben und Y—$NO_2$ oder Halogen darstellt, in Gegenwart eines dipolaren aprotischen oder protischen Lösungsmittels bei erhöhter Temperatur mit einer Verbindung der Formel III

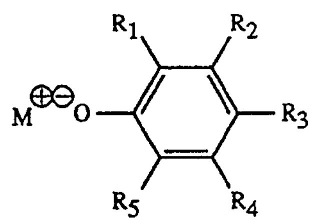

(III)

umsetzt, worin $R_1$ bis $R_5$ die in Anspruch 1 angegebenen Bedeutungen haben und $M^{\oplus}$ für ein Alkalimetallkation, ein quaternäres Ammonium— oder ein Phosphoniumkation steht.

Das erfindungsgemässe Verfahren wird bevorzugt bei Temperaturen von 50 bis 200°C, besonders 50 bis 150°C durchgefütrh. Die Salze der Formel III können als solche eingesetzt werden oder durch Umsetzung eines entsprechenden Phenols mit einer Alkalimetall—, Ammonium— oder Phosphoniumbase in situ im Reaktionsgemisch erzeugt werden. Die Salze der Formel III können in äquimolaren Mengen oder im Ueberschuss, z.B. bis zu 40 Mol-% Ueberschuss eingesetzt werden.

Geeignete Lösungsmittel sind z.B. N-substituierte Carbonsäureamide und Lactame (z.B. Dimethylformamid oder N-Methylpyrrolidon), Sulfoxide und Sulfone (z.B. Dimethylsulfoxid, Tetramethylensulfon) oder Ether (z.B. Di-n-propylether, Di-n-butylether, Tetrahydrofuran oder Dioxan).

Die Isolierung und Reinigung der Verbindungen der Formel I erfolgt nach üblichen Methoden, z.B. durch Kristallisation und Umkristallisation, oder chromatographische Verfahren.

Die Verbindungen der Formel III sind bekannt oder in bekannter Weise durch die Umsetzung entsprechender Phenole mit Alkalimetall—, Ammonium— oder Phosphoniumbasen erhältlich. Als Alkalimetallkationen kommen besonders $Li^{\oplus}$, $Na^{\oplus}$ und $K^{\oplus}$ in Frage ; geeignete quaternäre Oniumkationen sind z.B. $(C_1$-$C_6$-$Alkyl)_4N^{\oplus}$, $(C_1$ -$C_6$-Alkyl)$P^{\oplus}$ oder Tetraphenylphosphonium.

Verbindungen der Formel I mit funktionellen Gruppen wie z.B. —COOH oder —$NH_2$, können in bekannter Weise zu Estern oder Amiden derivatisiert werden.

Die Verbindungen der Formel II, worin Y als Halogen bevorzugt —F, —Cl oder —Br ist, sind teilweise bekannt oder nach an sich bekannten Verfahren durch intramolekulare Friedel-Crafts-Acylierung herstellbar (Thioxanthone siehe z.B. US-A-4,385,182 oder F. Mayer, Chem. Ber. 43, S. 584 ff (1910) ; Xanthone siehe z.B. F. Ullmann et al., Chem. Ber. 38, S. 2120 ff (1905) ; Acridone siehe z.B. C.W. Renocastle et al. Synthesis 1985, S. 21 ff). Thioxanthonsulfoxide und —sulfodioxide können auch durch Oxidation der Thioxanthone erhalten werden.

Die Verbindungen der Formel I sind hitzebeständig, farblos bis leicht gelb und überwiegend kristallin sowie

löslich in organischen Lösungsmitteln. Sie sind hochwirksame Photoinitiatoren und Photosensibilisatoren für photopolymerisierbare oder photodimerisierbare Systeme, die ethylenisch ungesättigte Doppelbindungen enthalten.

Bei Bestrahlung der Verbindungen, gegebenenfalls in einem Substrat, mit Licht einer Wellenlänge von etwa 300 bis 450 nm wird überraschend eine ausgeprägte Farbänderung nach blau bis violett gefunden. Diese Farbänderung ist irreversibel.

Die Verbindungen der Formel I können daher als Photoinitiatoren und besonders Photosensibilisatoren in photopolymerisierbaren Systemen verwendet werden, wobei sie gleichzeitig als Farbindiwikatoren wirken. So ist es möglich, belichtete Produkte (z.B. Schutzschichten, Druckplatten, Offsetdruckplatten, gedruckte Schaltungen, Lötstoppmasken) zu markieren und von unbelichteten Produkten zu unterscheiden, und ferner bei einer Produktekontrolle fehlerhaft belichtete Produkte vor oder nach der Entwicklung auszusondern.

Der erhebliche Vorteil bei der Verwendung als Farbindikatoren liegt in der Erhöhung der Sensibilisatorwirkung. Ueblicherweise als Farbumschlagsysteme eingesetzte Zusätze bewirken eine Erniedrigung der Photosensibilität.

Die Verbindungen der Formel I können auch als solche, in Lösung oder in Polymeren eingearbeitet, als Photofarbindikatoren oder als Photoschaltelemente verwendet werden.

Ein weiterer Gegenstand der Erfindung ist eine strahlungsempfindliche Zusammensetzung, enthaltend

a) ein strahlungsempfindliches organisches Material, und

b) mindestens eine Verbindung der Formel I, einschliesslich 1-Phenoxy-4-methylxanthen-9-on.

Die Verbindungen der Formel I können in einer Menge von 0,001 bis 20 Gew.-%, besonders 0,001 bis 10 Gew.-% und insbesondere 0,01 bis 5 Gew.-% enthalten sein, bezogen auf die Komponente a).

Strahlungsempfindliche und damit auch photostrukturierbare Materialien sind bekannt. Es kann sich um Positiv— oder Negativsysteme handeln. Solche Materialien sind z.B. von G.E. Green et al. in J. Macro. Sci. Revs. Macr. Chem., C21(2), 187-273 (1981-82) und von G.A. Delzenne in Adv. Photochem., 11, S. 1-103 (1979) beschrieben.

Vorzugsweise handelt es sich bei dem strahlungempfindlichen organischen Material um a1) eine nichtflüchtige monomere, oligomere oder polymere Substanz mit photopolymerisierbaren oder photodimerisierbaren ethylenisch ungesättigten Gruppen, a2) um ein kationisch härtbares System oder a3) um photovernetzbare Polyimide.

Photopolymerisierbare Substanzen sind z.B. Acryl— und besonders Methacrylsäureester von Polyolen, z.B. Ethylenglykol, Propandiol, Butandiol, Hexandiol, Di(hydroxymethyl)cyclohexan, 2,2-Bis(4-hydroxyethyloxyphenyl)-propan, Polyoxyalkylendiole wie z.B. Di—, Tri— oder Tetraethylenglykol, Di— oder Tri-1,2-propylenglykol, Trimethylol-methan, —ethan oder —propan und Pentaerythrit, die alleine, in Mischungen und in Abmischung mit Bindemitteln verwendet werden können. Geeignet sind auch die Anlagerungsprodukte von Acryl— und besonders Methacrylsäure an Bisglycidyloxyverbindungen, z.B. 2,2-Bis(4-glycidyloxyphenyl)-propan. Ferner ist Methylen-bis-acrylamid geeignet.

Photodimerisierbare Substanzen sind z.B. Homo— und Copolymere, die Zimtsäuregruppen oder substituierte Maleinimidylverbindungen in Seitengruppen oder Chalkongruppen in der Polymerkette enthalten.

Bevorzugt sind solche Zusammensetzungen, worin Komponente a1) ein Homo— oder Copolymer von Acryl—, Methacryl— oder Maleinsäureestern ist, deren Estergruppen einen Rest der Formel

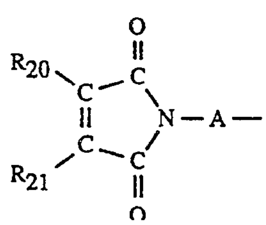

enthalten, worin A für unsubstituiertes oder mit Hydroxyl substituiertes lineares oder verzweigtes $C_2$-$C_{12}$-Alkylen, Cyclohexylen oder Phenylen steht, und $R_{20}$ und $R_{21}$ unabhängig voneinander Cl, Br, Phenyl oder $C_1$-$C_4$-Alkyl bedeuten, oder $R_{20}$ und $R_{21}$ zusammen Trimethylen, Tetramethylen oder

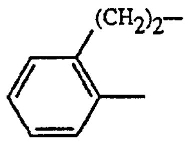

bedeuten. Solche Polymere sind z.B. in der US-A-4 193 927 beschrieben.

Die photopolymerisierbaren oder photodimerisierbaren Substanzen können weitere für die Verarbeitung oder Anwendung übliche Additive enthalten, sowie zusätzlich andere Photoinitiatoren oder Photosensibilisatoren.

Bei den kationisch härtbaren Systemen handelt es sich bevorzugt um Epoxidverbindungen mit mindestens 2 Epoxidgruppen im Molekül, denen ein Photoinitiator einverleibt ist. Geeignete Photoinitiatoren sind z.B. Metallocene, Metallocencarbonyle und Oniumsalze, die z.B. in den zuvor erwähnten Publikationen beschrieben sind. Die härtbaren Systeme können für die Verarbeitung und Anwendung übliche Zusatzstoffe enthalten.

Photoempfindliche Polyimide sind z.B. in der DE-A-1 962 588, EP-A-0 132 221, EP-A-0 134 752, EP-A-0 162 017, EP-A-0 181 837 und EP-A-0 182 745 beschrieben.

Die erfindungsgemässe Zusammensetzung wird nach bekannten Methoden als Schicht auf Substrate aufgebracht und entweder durch flächige Bestrahlung eine Schutzschicht oder durch Bestrahlung unter eine Photomaske und nachfolgende Entwicklung ein Reliefbild erzeugt.

Ein weiterer Gegenstand der Erfindung ist eine Zusammensetzung, enthaltend

a) ein farbloses organisches Lösungsmittel, ein Polymer oder ein organisches Glas oder ein Verbundglas, und

b) eine Verbindung der Formel I, einschliesslich 1-Phenoxy-4-methyl-xanthen-9-on. Die Komponente b) ist bevorzugt in einer Menge von 0,001 bis 20 Gew.-%, besonders 0,001 bis 10 Gew.-% und insbesondere 0,01 bis 5 Gew.-% enthalten, bezogen auf Komponente a). Organische Lösungen können zur Beschichtung von anderen Substanzen verwendet werden, z.B. anorganischen Gläsern, die dann als photoschaltbare Substrate verwendet werden können. Die Verbindungen der Formel I können auch auf Substrate aufsublimiert werden. Die beschichteten Substrate können mit einer Schutzschicht aus z.B. transparenten Polymeren versehen werden. Geeignete Lösungsmittel sind z.B. Kohlenwasserstoffe, halogenierte Kohlenwasserstoffe, Ketone, Carbonsäureester und Lactone, N-alkylierte Säureamide und Lactame, Alkanole und Ether.

Geeignete Polymere sind z.B. Duroplaste, Thermoplaste und strukturell vernetzte Polymere. Die Polymeren sind bevorzugt transparent. Solche Polymere und organische Gläser sind dem Fachmann geläufig. Die Einarbeitung der erfindungsgemässen Verbindungen erfolgt nach üblichen Methoden, z.B. mit Lösungsverfahren und Entfernen des Lösungsmittels, Kalandrieren oder Extrusion. Die erfindungsgemässen Verbindungen können den Substraten auch vor, während oder nach der Herstellung einverleibt werden.

Ein weiterer Gegenstand der Erfindung ist ein Verfahren zur Herstellung von gefärbten Materialien unter Einwirkung von Licht, dadurch gekennzeichnet, dass man dem Material eine Verbindung der Formel I einschliesslich 1-Phenoxy-4-methylxanthen-9-on einverleibt und darauf das Material mit Licht bestrahlt.

Ferner ist ein Gegenstand der Erfindung die Verwendung von Verbindungen der Formel I einschliesslich 1-Phenoxy-4-methylxanthen-9-on als Photosensibilisatoren und Farbindikatoren oder Photoschaltelemente bei Lichteinwirkung.

Die nachfolgenden Beispiele erläutern die Erfindung näher.

A) Herstellunpsbeispiele

Beispiele A1-A76 : 16 mmol 1-Halogenxanthon, —thioxanthon oder —acridon, 24 mmol Phenol, 40 mmol Kaliumcarbonat und 45 ml Lösungsmittel werden unter Rühren erwärmt. Die Reaktionsmischung wird in 200 ml Eis gegossen, der Niederschlag abfiltriert und in Toluol/Tetrahydrofuran (1 : 1) gelöst. Die Lösung wird nacheinander mit 5%-iger wässriger NaOH, Wasser und gesättigter wässriger Kochsalzlösung gewaschen und mit Natriumsulfat getrocknet. Das Lösungsmittel wird abdestilliert und der Rückstand aus Toluol/Isopropanol (1 : 1) umkristallisiert.

Weitere Angaben sind in Tabellen 1a und 1b aufgeführt. Die Angaben beziehen sich auf die Formel

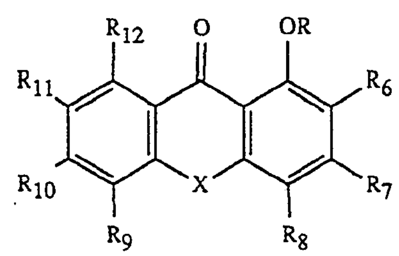

Tabelle 1a:

| Nr. A | X | R | $R_6$ | $R_7$ | $R_8$ | $R_9$ | $R_{10}$ | $R_{11}$ | $R_{12}$ | Schmelzpunkt (°C) |
|---|---|---|---|---|---|---|---|---|---|---|
| 1 | O | Ph | H | OPh | H | H | H | H | Cl | 168-176 |
| 2 | O | Ph | H | OPh | H | H | H | H | OPh | 181-183 |
| 3 | O | Ph | H | OPh | H | Cl | OPh | Cl | $CO_2C_2H_5$ | 195-197 |
| 4 | O | Ph | H | Cl | H | H | H | H | Cl | 152-154 |
| 5 | O | Ph | H | Cl | H | Cl | Cl | Cl | $CO_2C_2H_5$ | 229-231 |
| 6 | N-3,5-$F_2$Ph | Ph | H | F | H | H | H | H | H | |
| 7 | $NC_2H_5$ | Ph | H | Cl | H | H | H | H | H | 189-191 |
| 8 | $NCH_2Ph$ | Ph | H | F | H | H | H | H | H | 223-225 |
| 9 | $NCH_3$ | Ph | H | OPh | H | H | H | H | H | 100-105 |
| 10 | $NCH_3$ | Ph | H | H | H | H | H | H | $CF_3$ | 142-145 |
| 11 | N-2-COOH-Ph | Ph | H | Cl | H | H | H | H | H | <260 |
| 12 | $NC_2H_5$ | 3-Br-Ph | H | Cl | H | H | H | H | H | 183-187 |
| 13 | $NC_2H_5$ | 4-Cl-Ph | H | Cl | H | H | H | H | H | 147-148 |
| 14 | $NC_2H_5$ | Ph | H | PhO | H | H | H | H | H | 164-65 |
| 15 | $NC_2H_5$ | 3-Br-Ph | H | 3-BrPhO | H | H | H | H | H | 126-129 |
| 16 | $NCH_3$ | Ph | H | H | Cl | H | H | H | H | 129-130 |

Tabelle 1a:

| Nr. A | X | R | $R_6$ | $R_7$ | $R_8$ | $R_9$ | $R_{10}$ | $R_{11}$ | $R_{12}$ | Schmelzpunkt (°C) |
|---|---|---|---|---|---|---|---|---|---|---|
| 17 | $NC_2H_5$ | 3,5-$(CH_3O)_2$Ph | H | 3,5-$(CH_3O)_2$PhO | H | H | H | H | H | 152-154 |
| 18 | $NC_2H_5$ | 3,5-$(CH_3O)_2$Ph | H | Cl | H | H | H | H | H | 180-183 |
| 19 | $NC_2H_5$ | 4-Cl-Ph | H | 4-ClPhO | H | H | H | H | H | 148-149 |
| 20 | $NC_2H_5$ | 4-(Oct-2-yl-$O_2$C)Ph | H | Cl | H | H | H | H | H | Oel |
| 21 | $NC_2H_5$ | 4-(Oct-2-yl-$O_2$C)Ph | H | 4-(Oct-2-yl-$O_2$C)PhO | H | H | H | H | H | Oel |
| 22 | $NC_2H_5$ | 4-$(C_2H_5O_2C)$Ph | H | Cl | H | H | H | H | H | 138-140 |
| 23 | N-i-Propyl | Ph | H | Cl | H | H | H | H | H | 143-145 |
| 24 | $NCHCH_3$ (mit $CO_2C_2H_5$ an CH) | Ph | H | Cl | H | H | H | H | H | 144-147 |
| 25 | $NCHCH_3$ (mit $CO_2C_2H_5$ an CH) | Ph | H | OPh | H | H | H | H | H | 60-70 |
| 26 | $NCH_2$-CH-$nC_4H_9$ (mit $C_2H_5$ an CH) | Ph | H | Cl | H | H | H | H | H | 102-104 |
| 27 | $NCH_2$-CH-$nC_4H_9$ (mit $C_2H_5$ an CH) | Ph | H | OPh | H | H | H | H | H | 102-105 |
| 28 | $NC_2H_5$ | Ph | H | Cl | H | H | H | H | H | 128-130 |
| 29 | $NCH_3$ | Ph | Cl | H | Cl | H | H | H | H | 173-174 |
| 30 | $NC_3H_7$ | Ph | Cl | H | Cl | H | H | H | H | 116-118 |
| 31 | S | Ph | Cl | Cl | Cl | H | H | H | H | 190-192 |
| 32 | S | Ph | Cl | PhO | Cl | H | H | H | H | 203-204 |

Tabelle 1a:

| Nr. A | X | R | $R_6$ | $R_7$ | $R_8$ | $R_9$ | $R_{10}$ | $R_{11}$ | $R_{12}$ | Schmelz-punkt (°C) |
|---|---|---|---|---|---|---|---|---|---|---|
| 33 | S | Ph | Br | H | F | H | H | H | H | 199-202 |
| 34 | S | Ph | H | H | $CH_3O$ | H | H | H | H | 191-193 |
| 35 | S | Ph | Cl | H | Cl | H | H | H | H | 183-185 |
| 36 | S | 4-$NO_2$-Ph | Cl | H | Cl | H | H | H | H | 228-230 |
| 37 | S | 4-F-Ph | Cl | H | Cl | H | H | H | H | 149-150 |
| 38 | S | 4-Br-Ph | Cl | H | Cl | H | H | H | H | 217-219 |
| 39 | S | 4-Cl-Ph | Cl | H | Cl | H | H | H | H | 192-195 |
| 40 | S | 4-J-Ph | Cl | H | Cl | H | H | H | H | 236-238 |
| 41 | S | 4-$CH_3$-Ph | Cl | H | Cl | H | H | H | H | 175-177 |
| 42 | S | 4-$CH_3O$-Ph | Cl | H | Cl | H | H | H | H | 153-155 |
| 43 | S | $(C_2H_5)_2N$-Ph | Cl | H | Cl | H | H | H | H | 134-135 |
| 44 | S | 3-Cl-Ph | Cl | H | Cl | H | H | H | H | 150-152 |
| 45 | S | 3-$CH_3O$-Ph | Cl | H | Cl | H | H | H | H | 169-171 |
| 46 | S | 3,5-$(CH_3O)_2$-Ph | Cl | H | Cl | H | H | H | H | 192-194 |
| 47 | S | 2,4-$Cl_2$-Ph | Cl | H | Cl | H | H | H | H | 235-237 |

Tabelle 1a:

| Nr. A | X | R | $R_6$ | $R_7$ | $R_8$ | $R_9$ | $R_{10}$ | $R_{11}$ | $R_{12}$ | Schmelz-punkt (°C) |
|---|---|---|---|---|---|---|---|---|---|---|
| 48 | S | 3-Br-Ph | Cl | H | Cl | H | H | H | H | 172-174 |
| 49 | S | 3,4-$Cl_2$-Ph | Cl | H | Cl | H | H | H | H | 187-188 |
| 50 | S | Ph | Cl | H | Cl | H | H | $CO_2C_2H_5$ | H | 178-183 |
| 51 | S | 4-CN-Ph | Cl | H | Cl | H | H | H | H | 248-251 |
| 52 | S | 4-($C_2H_5O_2C$)-Ph | Cl | H | Cl | H | H | H | H | 172-174 |
| 53 | S | 4-t-Octyl-Ph | Cl | H | Cl | H | H | H | H | 137-139 |
| 54 | S | 4-(n-$C_{12}H_{25}O_2C$)-Ph | Cl | H | Cl | H | H | H | H | 109-112 |
| 55 | S | Ph | Cl | H | Cl | H | $C_2H_5O_2C$ | H | H | 170-173 |
| 56 | S | Ph | $CH_3$ | H | $CH_3$ | H | H | H | $C_2H_5O_2C$ | 153-155 |
| 57 | S | 4-(n-$C_{12}H_{25}O_2C$)-Ph | Cl | H | Cl | H | $C_2H_5O_2C$ | H | H | 129-133 |
| 58 | S | 3-($C_2H_5O_2C$)-Ph | Cl | H | Cl | H | H | H | H | 163-165 |
| 59 | S | 3-$CF_3$-Ph | Cl | H | Cl | H | H | H | H | 167-169 |
| 60 | S | Ph | Cl | H | Cl | H | H | H | $C_2H_5O_2C$ | 168-170 |
| 61 | S | Ph | Cl | H | Cl | H | H | H | $C_2H_5CHO_2C$<br>n-$C_5H_{11}$ | 127-129 |
| 62 | S | 4-(n-$C_{12}H_{25}O_2C$)-Ph | Cl | H | Cl | H | H | H | $C_2H_5CHO_2C$<br>n-$C_5H_{11}$ | Oel |

Tabelle 1a:

| Nr. A | X | R | $R_6$ | $R_7$ | $R_8$ | $R_9$ | $R_{10}$ | $R_{11}$ | $R_{12}$ | Schmelzpunkt (°C) |
|---|---|---|---|---|---|---|---|---|---|---|
| 63 | S | 1-Naphthyl | Cl | H | Cl | H | H | H | H | 247-249 |
| 64 | S | 2-Naphthyl | Cl | H | Cl | H | H | H | H | 197-200 |
| 65 | S | 3-OH-4-R'-Ph | Cl | H | Cl | H | H | H | H | 235-238 |
| 66 | S | Ph | H | Cl | H | H | H | H | H | 114-116 |
| 67 | S | Ph | H | PhO | H | H | H | H | H | 151-153 |
| 68 | S | 2,4-$Cl_2$-Ph | H | Cl | H | H | H | H | H | 205-208 |
| 69 | S | 3,5-$Cl_2$-Ph | H | Cl | H | H | H | H | H | 195-197 |
| 70 | S | 3,5-$Cl_2$-Ph | H | 3,5-$Cl_2$-Ph | H | H | H | H | H | 202-204 |
| 71 | S | 3,5-$Cl_2$-Ph | Cl | H | Cl | H | H | H | H | 214-218 |
| 72 | S | Ph | H | Cl | Cl | H | H | H | H | 162-165 |
| 73 | S | 3-$CH_3O$-Ph | H | Cl | H | H | H | H | H | 102-104 |
| 74 | S | 3-$CH_3O$-Ph | Cl | Cl | Cl | H | H | H | H | 172-174 |
| 75 | S | 3-$CH_3O$-Ph | Cl | 3-$CH_3O$-Ph- | Cl | H | H | H | H | — |
| 76 | S | Ph | Br | H | Br | H | H | H | H | 174-176 |

Tabelle 1b:

| Nr. A | 1-Halogen | Lösungsmittel | Reaktionstemperatur (°C) | Reaktionszeit (h) | Ausbeute (%) |
|---|---|---|---|---|---|
| 1 | Cl | DMSO | 120 | 19 | 4 |
| 2 | Cl | DMSO | 120 | 19 | 7 |
| 3 | Cl | DMSO | 120 | 18 | 53 |
| 4 | Cl | $Bu_2O$ | 120 | 23 | 52 |
| 5 | Cl | $Bu_2O$ | 130 | 21 | 44 |
| 6 | F | Dioxan | 100 | 3 | 33 |
| 7 | Cl | $Bu_2O$ | 150 | 72 | 84 |
| 8 | F | Dioxan | 100 | 4 | 76 |
| 9 | Cl | DMSO | 120 | 2 | 78 |
| 10 | F | DMSO | 100 | 36 | 45 |
| 11 | Cl | DMSO | 130 | 18 | 58 |
| 12 | Cl | $Bu_2O$ | 140 | 24 | 72 |
| 13 | Cl | $Bu_2O$ | 140 | 16 | 66 |
| 14 | Cl | DMSO | 140 | 23 | 80 |
| 15 | Cl | DMSO | 140 | 23 | 75 |
| 16 | Cl | DMSO | 130 | 24 | 71 |
| 17 | Cl | DMSO | 140 | 18 | 52 |
| 18 | Cl | $Bu_2O$ | 140 | 64 | 24 |
| 19 | Cl | DMSO | 140 | 19 | 72 |
| 20 | Cl | $Bu_2O$ | 140 | 40 | 95 |
| 21 | Cl | DMSO | 140 | 61 | 54 |
| 22 | Cl | $Bu_2O$ | 140 | 42 | 31 |
| 23 | Cl | DMSO | 140 | 45 | 64 |
| 24 | Cl | $Bu_2O$ | 140 | 5 Tage | 21 |
| 25 | Cl | $Bu_2O$ | 140 | 5 Tage | 15 |

Tabelle 1b:

| Nr. A | 1-Halogen | Lösungsmittel | Reaktionstemperatur (°C) | Reaktionszeit (h) | Ausbeute (%) |
|---|---|---|---|---|---|
| 26 | Cl | $Bu_2O$ | 140 | 68 | 61 |
| 27 | Cl | $Bu_2O$ | 140 | 68 | 8 |
| 28 | Cl | $Bu_2O$ | 140 | 69 | 53 |
| 29 | Cl | NMP | 100 | 18 | 93 |
| 30 | Cl | DMSO | 110 | 3 | 83 |
| 31 | Cl | Dioxan | 120 | 24 | 44 |
| 32 | Cl | Dioxan | 120 | 24 | 13 |
| 33 | F | $Bu_2O$ | 130 | 17 | 75 |
| 34 | F | DMSO | 130 | 9 | 36 |
| 35 | Cl | DMSO | 90 | 23 | 58 |
| 36 | Cl | DMSO | 120 | 23 | 17 |
| 37 | Cl | DMSO | 90 | 23 | 58 |
| 38 | Cl | DMSO | 80 | 28 | 55 |
| 39 | Cl | DMSO | 80 | 28 | 51 |
| 40 | Cl | DMSO | 80 | 17 | 48 |
| 41 | Cl | DMSO | 80 | 17 | 58 |
| 42 | Cl | DMSO | 80 | 19 | 65 |
| 43 | Cl | DMSO | 100 | 20 | 34 |
| 44 | Cl | DMSO | 80 | 21 | 61 |
| 45 | Cl | DMSO | 100 | 6 | 55 |
| 46 | Cl | DMSO | 100 | 18 | 64 |
| 47 | Cl | DMSO | 120 | 24 | 47 |
| 48 | Cl | DMSO | 80 | 18 | 60 |
| 49 | Cl | DMSO | 100 | 18 | 56 |
| 50 | Cl | DMSO | 80 | 3 | 70 |

Tabelle 1b:

| Nr. A | 1-Halogen | Lösungs-mittel | Reaktions-temperatur (°C) | Reaktions-zeit (h) | Ausbeute (%) |
|---|---|---|---|---|---|
| 51 | Cl | DMSO | 100 | 19 | 58 |
| 52 | Cl | DMSO | 100 | 15 | 61 |
| 53 | Cl | DMSO | 80 | 16 | 53 |
| 54 | Cl | DMSO | 100 | 18 | 49 |
| 55 | Cl | DMSO | 80 | 18 | 55 |
| 56 | Br | DMSO | 140 | 32 | 3 |
| 57 | Cl | DMSO | 90 | 22 | 57 |
| 58 | Cl | DMSO | 80 | 22 | 56 |
| 59 | Cl | DMSO | 90 | 6 | 61 |
| 60 | Cl | DMSO | 80 | 16 | 67 |
| 61 | Cl | DMSO | 80 | 6 | 65 |
| 62 | Cl | DMSO | 100 | 6 | 88 |
| 63 | Cl | DMSO | 100 | 22 | 52 |
| 64 | Cl | DMSO | 100 | 22 | 58 |
| 65 | Cl | DMSO | 100 | 18 | 73 |
| 66 | Cl | Dioxan | 100 | 47 | 63 |
| 67 | Cl | DMSO | 120 | 20 | 97 |
| 68 | Cl | Dioxan | 100 | 72 | 27 |
| 69 | Cl | Dioxan | 100 | 72 | 80 |
| 70 | Cl | DMSO | 120 | 18 | 70 |
| 71 | Cl | DMSO | 100 | 3 | 46 |
| 72 | Cl | $Bu_2O$ | 130 | 96 | 42 |
| 73 | Cl | Dioxan | 100 | 72 | 52 |
| 74 | Cl | $Bu_2O$ | 140 | 7 | 80 |

Tabelle 1b:

| Nr. A | 1-Halogen | Lösungs-mittel | Reaktions-temperatur (°C) | Reaktions-zeit (h) | Ausbeute (%) |
|---|---|---|---|---|---|
| 75 | Cl | — | — | — | 4[1)] |
| 76 | Br | $Bu_2O$ | 140 | 20 | 69 |

Abkürzungen :
DMSO : Dimethylsulfoxid
$Bu_2O$ : Di-(n-butyl)ether
NMP : N-Methylpyrrolidon
Ph : Phenyl

R' (Beispiel 65): 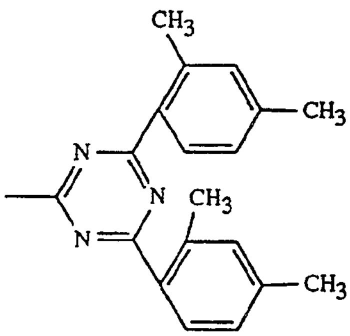

[1)] Nebenprodukt von Beispiel A74

Beispiele A77-A82 : 3-Methoxy-1-phenoxythioxanthon

340 mg (1 mmol) 3-Chlor-1-phenoxythioxanthon, 3 ml (75 mmol) Methanol und 420 mg (3 mmol) Kaliumcarbonat werden in 5 ml Dimethylsulfoxid 24 h auf 110°C erhitzt. Die Reaktionsmischung wird in Wasser gegossen, mit verdünnter Salzsäure neutralisiert und das Produkt mit Toluol/Tetrahydrofuran extrahiert. Die organische Phase wird mit gesättigter wässriger Natriumhydrogencarbonatlösung gewaschen, mit Natriumsulfat getrocknet und eingedampft. Der Rückstand wird chromatographisch (Kieselgel, Hexan/Essigester 10 : 1) gereinigt und anschliessend aus Toluol umkristallisiert. Ausbeute : 113 mg (34%), Schmelzpunkt 154-156°C.

Analog werden, unter Verwendung von Na-Phenylsulfinat, Thiophenol, Na-Methylmercaptid bzw. Thiosalicylsäure, die folgenden Verbindungen hergestellt (Tabelle 2) :

| Beispiel Nr. A | X | R | $R_6$ | $R_7$ | $R_8$ | $R_9$ | $R_{10}$ | $R_{11}$ | $R_{12}$ | Ausbeute (%) | Schmelz-punkt (°C) |
|---|---|---|---|---|---|---|---|---|---|---|---|
| 78 | S | Ph | H | $-SO_2Ph$ | H | H | H | H | H | 80 | 201-204 |
| 79 | S | Ph | H | -SPh | H | H | H | H | H | 78 | 161-163,5 |
| 80 | S | Ph | H | $-SCH_3$ | H | H | H | H | H | 5 | 173-176 |
| 81 | S | Ph | H | 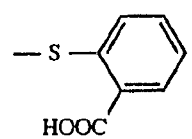 | Cl | H | H | H | H | 46 | 247-250 |
| 82 | S | Ph | H | 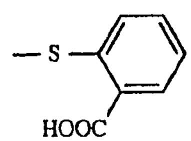 | H | H | H | H | H | 50 | 150-152 |

Beispiel A83 : N-Methyl-1-phenoxy-4-pyrrolylacridon

1,3 g (4 mmol) 4-Amino-N-methyl-1-phenoxyacridon und 0.8 g (6 mmol) 2,5-Dimethoxytetrahydrofuran werden in 10 ml Eisessig 30 min auf 130°C erhitzt. Danach wird die Reaktionsmischung in 100 ml Wasser gegossen und das Produkt mit Toluol/Tetrahydrofuran extrahiert. Die organische Phase wird nacheinander mit gesättigter wässriger Natriumhydrogencarbonat-Lösung, Wasser und gesättigter wässriger Kochsalz-Lösung gewaschen, mit Natriumsulfat getrocknet und eingedampft. Die säulenchromatographische Reinigung (Kieselgel, Ether/Hexan 1 : 1) und anschliessende Umkristallisation aus Toluol/Isopropanol ergibt das gewünschte Produkt. Weitere Angaben sind in Tabelle 3.

Beispiele A84-A86 : Analog Beispiel A83 werden die Verbindungen der Beispiele A84-A86 hergestellt, wobei im Beispiel A85 2,3-Dimethylbernsteinsäureanhydrid und in Beispiel A86 Dimethylmaleinsäureanhydrid anstelle von 2,5-Dimethoxytetrahydrofuran verwendet werden. Weitere Angaben sind in Tabelle 3.

Tabelle 3:

| Beispiel Nr. A | X | R | $R_6$ | $R_7$ | $R_8$ | $R_9$ | $R_{10}$ | $R_{11}$ | $R_{12}$ | Ausbeute (%) | Schmelzpunkt (°C) |
|---|---|---|---|---|---|---|---|---|---|---|---|
| 83 | $NCH_3$ | Ph | H | H | 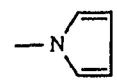 | H | H | H | H | 87 | 177-180 |
| 84 | S | Ph | H | H | 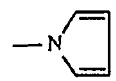 | H | H | H | H | 64 | 164-166 |
| 85 | $NCH_3$ | Ph | H | H | 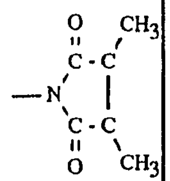 | H | H | H | H | 85 | 221-223 |
| 86 | $NCH_3$ | Ph | H | H | 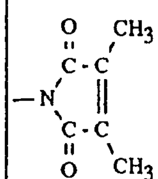 | H | H | H | H | 79 | 248-249 |

Beispiel A87 : 4-Acetylamino-N-methyl-1-phenoxyacridon

Eine Lösung von 0,7 g (2 mmol) 4-Amino-N-methyl-1-phenoxyacridon in 20 ml Chloroform wird mit 1,3 ml (16 mmol) Pyridin und 0,28 ml (4 mmol) Acetylchlorid versetzt und 6 h bei Raumtemperatur gerührt. Die Lösung wird mit Salzsäure (18%), Wasser und gesättigter wässriger Kochsalzlösung gewaschen, mit Natriumsulfat getrocknet und eingedampft. Der Rückstand wird mit Diethylether gewaschen und abfiltriert. Ausbeute : 0,6 g (83%) Schmelzpunkt : 252-254°C.

Beispiele A88 und A89 : Auf analoge Weise wie in Beispiel A87 werden erhalten : A88 : 4-(2-Ethylhexanoylamino)-N-methyl-1-phenoxy-acridon, Schmelzpunkt : 154-156°C, Ausbeute 70%.

A89 : 4-(3-Chlor-2,2-dimethylpropionylamino)-N-methyl-1-phenoxy-acridon, Schmelzpunkt : 217-218°C, Ausbeute : 80%.

Beispiel A90 : 2,4-Dichlor-1-phenoxythioxanthonsulfoxid

Eine Lösung von 3 g (8 mmol) 2,4-Dichlor-1-phenoxythioxanthon und 0,9 g (8 mmol) $H_2O_2$ (30%) in 100 ml Eisessig wird 1 h am Rückfluss erhitzt. Anschliessend wird in 250 ml Wasser gegossen und mit 150 ml Toluol extrahiert. Die organische Phase wird mit Wasser, wässrigem Natriumhydrogencarbonat-Lösung (10%) und gesättigter wässriger Kochsalzlösung gewaschen, mit Natriumsulfat getrocknet und eingedampft. Der Rückstand wird aus Toluol/Isopropanol umkristallisiert. Ausbeute 2,1 g (67%) Schmelzpunkt : 207-209°C.

Beispiel A91 : 2,4-Dichlor-1-phenoxythioxanthonsulfodioxid

Führt man die Reaktion mit 2,7 g $H_2O_2$ (24 mmol) (30%) aus und reinigt säulenchromatographisch (Kieselgel, $CH_2Cl_2$), so erhält man 3,26 g (74%) des Sulfons ; Schmelzpunkt : 197-198°C.

Beispiel A92 : 1-(3′,5′-Dichlorphenoxy)-3-(α-cyanobenzyl)-thioxanthon

2 g (4,9 mmol) 1-(3′,5′-Dichlorphenoxy)-3-chlor-thioxanthon, 0,86 g Benzylcyanid, 2,03 g Kaliumcarbonat und 20 ml DMSO werden während 6 Stunden bei 80°C gerührt. Das Gemisch wird auf 2N HCl/Tetrahydrofuranl/Toluol ausgetragen, die organische Phase abgetrennt, über Natriumsulfat getrocknet und eingedampft. Durch Chromatographie mit Methylenchlorid über Kieselgel werden 0,72 g (30%) Produkt erhalten, Schmelzpunkt 145-155°C.

Beispiel A93 : 1-(3′,5′-Dichlorphenoxy)-3-benzoyl-thioxanthon

0,3 g (0,61 mmol) der Verbindung gemäss Beispiel A92 werden mit 130 mg (0,92 mmol) Kaliumcarbonat und 5 ml DMSO bei 60°C gerührt. In das Gemisch wird während 6 Stunden bei 60°C Luft eingeleitet. Das Gemisch wird wie in Beispiel A92 aufgearbeitet und das Rohprodukt aus Methylenchlorid/Pentan umkristallisiert. Ausbeute 0,20 g (69%), Schmelzpunkt 160-163°C.

Beispiel A94 : 1-Phenoxy-thioxanthon-3,4-N-butylimid

3,0 g (7,8 mmol) 1-Nitro-thioxanthon-3,4-N-butylimid, 0,89 g (9,4 mmol) Phenol, 2,16 g (15,7 mmol) Kaliumcarbonat und 30 ml DMSO werden 2 Stunden bei 25°C gerührt. Das Gemisch wird auf 2N HCl/Methylenchlorid ausgetragen. Die organische Phase wird abgetrennt, über Natriumsulfat getrocknet und eingedampft. Aus Methylenchlorid/Pentan umkristallisiert, werden 2,88 g (86%) Produkt vom Schmelzpunkt 198-199°C erhalten.

Beispiele A95 und A96 : Analog Beispiel A94 werden, unter Verwendung der entsprechenden Phenole, die folgenden Verbindungen erhalten :

A95 : 1-(3,5-Dichlorphenoxy)-thioxanthon-3,4-N-butylimid, Schmelzpunkt 207-213°C, Ausbeute 81%.

A96 : 1-(2-Methoxy-4-formylphenoxy)-thioxanthon-3,4-N-butylimid, Schmelzpunkt 260-263°C, Ausbeute 78%.

Beispiel A97 : 2,4-Dichlor-1-(3-hydroxyphenyl)-N-methylacridon

8 g (25,6 mMol) N-Methyl-1,2,4-trichloracridon, 8,46 g (76,8 mMol) Resorcin, 14,15 g (102,4 mMol) Kaliumcarbonat und 80 ml NMP werden während 30 Stunden bei 60°C gerührt. Das Gemisch wird auf Wasser/Tetrahydrofuran/Toluol ausgetragen. Die organische Phase wird mit Wasser gewaschen, über Natriumsulfat getrocknet, über Kieselgel filtriert und eingedampft. Der Rückstand wird mit Methylenchlorid über Kieselgel chromatographiert. Die das Produkt enthaltenden Fraktionen werden eingedampft und der Rückstand aus o-Dichlorbenzol umkristallisiert. Ausbeute : 5,45 g (55%), Schmelzpunkt 220-222°C.

B) Anwendungsbeispiele

Beispiel B1 : 1,8 g Verbindung gemäss Beispiel A6 werden in 60 g Methacrylsäuremethylester gelöst und mit 3,6 mg Azisobutylonitril versetzt. Die Lösung wird unter Luftausschluss mit einer Spritze in eine Küvette übergeführt, die aus zwei Glasplatten mit einem PVC-Schlauch mit 3 mm Aussendurchmesser als Abstandshalter besteht. Nach 18 Stunden bei 70°C in einem Umluftofen ist der Methacrylsäuremethylester vollständig polymerisiert. Die so erhaltene klare Platte wird in Streifen geschnitten und für Belichtungsversuche eingesetzt. Bei Belichtung unter einer Maske mit einer 5000 W Quecksilberhochdrucklampe bei 80 cm Abstand wird ein tiefblaues negatives Abbild der Maske erhalten.

Beispiele B2-B5 : Farbumschlapssysteme als Lichtindikatoren, Photosensibilisatoren und —initiatoren

Lösungen bestehend aus 100 g technischem Epoxidkresolnovolak (Epoxidwert 4,5 Aeq/kg), 50 g eines

technischen Bisphenol-A-diglycidylethers (Epoxidwert 0,35 Aeq/kg), 30 g Talkum Ultramix (Cyprus), 180 g Cyclohexanon, $5 \times 10^{-3}$ mol der in Tabelle 4 aufgeführten erfindungsgemässen Verbindungen und 4 g ($\eta^6$-Cumol)($\eta^5$-cyclopentadienyl)-eisen(II)-trifluormethansulfonat werden mit einem Drahtrakel auf eine Leiterplatte aufgebracht. Der vorerst nasse Film wird bei 80°C getrocknet. Die Belichtung der so hergestellten Platten erfolgt mit einer 5000 Watt Quecksilberhochdrucklampe im Abstand zur aufgelegten Maske (Stouffer-Keil) von 50 cm. Die Belichtungszeit beträgt 1 Minute. Danach wird die Platte 10 Minuten bei 110°C vorgehärtet. Die Entwicklung erfolgt in Cyclohexanon, wobei die nicht belichteten Anteile (Lötaugen) gelöst werden. Danach werden die Platten 30 Minuten bei 135°C nachgehärtet. Die so erhaltenen Platten werden 10 Sekunden in ein Lötbad (270°C) eingetaucht), wobei keine Veränderungen festgestellt werden.

Die Farbumschläge und die letzte abgebildete Keilstufe des Stoufferkeils (LKS) sind in Tabelle 4 angegeben.

Tabelle 4:

| Beispiel Nr. | Verbindung gemäss Beispiel | Farbumschlag | LKS |
|---|---|---|---|
| B2 | A69 | Farblos / Grünblau | 7 |
| B3 | A70 | Farblos / Violett | 8 |
| B4 | A7 | Farblos / Violett | 10 |
| B5 | A93 | Farblos / Grün | 10 |

Beispiele B6-B74 :

Die Eignung dieser Materialien wird in 2 Formulierungen von lichtempfindlichen Offsetplattenbeschichtungen geprüft :

Formulierung I

enthaltend nur die erfindungsgemässen Verbindungen und keinen zusätzlichen Sensibilisator.

Sie besteht aus :

1) 2,50 g Copolymer aus 86 Gewichtsteilen 6-(Dimethylmaleinimidyl)hex-1-yl-methacrylsäureester und 14 Gewichtsteilen Methacrylsäure (40 Gew.-% in Methoxypropanol), $\overline{M}_w$ : 100000
2) 5,00 ml Erfindungsgemässe Verbindung (1 Gew.-% in Dimethylformamid)
3) 0,50 ml Rhodamin B (Farbstoff), 1 Gew.-% in Ethylcellulose
4) 2,50 ml Ethylglycolacetat

Formulierung II

enthält neben den erfindungsgemässen Verbindungen noch einen zusätzlichen, handelsüblichen Sensibilisator.

1) 2,50 g Wie in Formulierung I
2) 2,50 ml Erfindungsgemässe Verbindung (2 Gew.-% in Dimethylformamid)
3) 2,50 ml 3,4-Thioxanthondicarbonsäuredioctylester, 2 Gew.-% in Dimethylformamid
4) 0,50 ml Rhodamin B, 1 Gew.-% in Ethylcellulose
5) 2,50 ml Ethylglycolacetat

Die Formulierungen werden mittels Schleuderbeschichtung (30 sec. bei 500 U/min) auf ein Offsetplattensubstrat, d.h. elektrolytisch aufgerauhtes und anodisiertes Aluminiumblech aufgetragen und 15 min bei 80°C getrocknet. Es resultiert ein Beschichtungsgewicht von 1,1-1,5 g/m². Danach wird mit einer 2000 Watt Metallhalogenlampe während 100 sec (Abstand 75 cm von Vakuumrahmen) durch einen Stouffer-Keil (Inkremente der optischen Dichte O.D. = 0,15) belichtet. Die Entwicklung erfolgt manuell bei 20°C durch leichtes Reiben während 45 sec. mit einem Tampon, das mit folgender Entwicklerlösung getränkt ist :

75,0 g Natrium-metasilicat-pentahydrat
0,4 g Netzmittel (Supronic B 50, ABM Chemicals Ltd.)
925,0 g Deionisiertes Wasser.

Nach kurzem Spülen mit Leitungswasser lässt man die Platten trocknen und beurteilt danach die Lichtempfindlichkeit durch Bestimmung der letzten noch abgebildeten Keilstufe des numerierten Stoufferkeils (LKS).

Je mehr Keilstufen mit einer bestimmten Belichtungsenergie abgebildet werden, desto höher ist die Photoempfindlichkeit der Schicht.

Praktisch alle der hier verwendeten Formulierungen ergeben Offsetdruckplatten von hoher Lichtempfindlichkeit und guter Abriebfestigkeit, was eine hohe Auflagenstärke erlaubt.

Die Resultate sind in Tabelle 5 zusammengefasst. Die Güte des Farbumschlags direkt nach der Belichtung wird mit einer Note versehen :

1 = Bild schwach sichtbar

2 = Bild gut sichtbar

3 = Bild sehr gut sichtbar.

Ebenfalls beurteilt wurde die zeitliche Dauer des Bildes (Bildstabilität), indem nach 1 h und nach 24 h eine qualitative Kontrolle über eventuelles Verblassen des Bildeindrucks erfolgt.

Tabelle 5:

| Beispiel Nr. | Verbindung von Beispiel Nr. | Formulierung I | | | Formulierung II | | |
|---|---|---|---|---|---|---|---|
| | | LKS | Farbumschlag | Bildstabilität (h) | LKS | Farbumschlag | Bildstabilität (h) |
| B6 | A92 | 10 | 3 | 1 | 10 | 3 | 1 |
| B7 | A9 | 7 | 2 | 24 | 9 | 2 | 24 |
| B8 | A67 | 5 | 1 | 24 | 8 | 1 | 24 |
| B9 | A95 | 10 | 3 | 24 | 10 | 3 | 24 |
| B10 | A68 | 7 | 1 | 24 | 9 | 1 | 24 |
| B11 | A69 | 7 | 1 | 24 | 8 | 1 | 24 |
| B12 | A70 | 6 | 1 | 24 | 8 | 1 | 24 |
| B13 | A71 | 9 | 2 | 24 | 9 | 2 | 24 |
| B14 | A92 | 11 | 2 | 24 | 9 | 2 | 24 |
| B15 | A66 | 7 | 1 | 24 | 10 | 1 | 24 |
| B16 | A78 | 9 | 2 | 24 | 9 | 2 | 24 |
| B17 | A79 | 7 | 2 | 24 | 9 | 2 | 24 |
| B18 | A80 | 3 | 1 | 24 | 7 | 1 | 24 |
| B19 | A73 | 6 | 1 | 24 | 9 | 2 | 24 |
| B20 | A74 | 5 | 2 | 24 | 9 | 2 | 24 |

Tabelle 5:

| Beispiel Nr. | Verbindung von Beispiel Nr. | Formulierung I | | | Formulierung II | | |
|---|---|---|---|---|---|---|---|
| | | LKS | Farbumschlag | Bildstabilität (h) | LKS | Farbumschlag | Bildstabilität (h) |
| B21 | A35 | 8 | 2 | 24 | 10 | 2 | 24 |
| B22 | A36 | 7 | 1 | 24 | 10 | 1 | 24 |
| B23 | A37 | 7 | 2 | 24 | 9 | 2 | 24 |
| B24 | A38 | 7 | 2 | 24 | 9 | 2 | 24 |
| B25 | A39 | 6 | 2 | 24 | 9 | 2 | 24 |
| B26 | A40 | 6 | 2 | 24 | 9 | 2 | 24 |
| B27 | A41 | 6 | 2 | 24 | 8 | 2 | 24 |
| B28 | A42 | 0 | 1 | 24 | 7 | 1 | 24 |
| B29 | A43 | 0 | 1 | 24 | 5 | 1 | 24 |
| B30 | A44 | 7 | 2 | 24 | 9 | 2 | 24 |
| B31 | A45 | 6 | 2 | 24 | 8 | 2 | 24 |
| B32 | A46 | 6 | 2 | 24 | 8 | 2 | 24 |
| B33 | A47 | 7 | 2 | 24 | 8 | 2 | 24 |
| B34 | A48 | 7 | 2 | 24 | 9 | 2 | 24 |
| B35 | A49 | 8 | 2 | 24 | 9 | 2 | 24 |

Tabelle 5:

| Beispiel Nr. | Verbindung von Beispiel Nr. | Formulierung I | | | Formulierung II | | |
|---|---|---|---|---|---|---|---|
| | | LKS | Farbumschlag | Bildstabilität (h) | LKS | Farbumschlag | Bildstabilität (h) |
| B36 | A6 | 8 | 3 | 24 | 9 | 3 | 24 |
| B37 | A34 | 4 | 1 | 1 | 7 | 1 | 1 |
| B38 | A33 | 6 | 2 | 24 | 8 | 2 | 24 |
| B39 | A84 | 6 | 1 | 24 | 8 | 1 | 24 |
| B40 | A34 | 9 | 2 | 24 | 9 | 2 | 24 |
| B41 | A50 | 6 | 2 | 24 | 8 | 2 | 24 |
| B42 | A51 | 8 | 2 | 24 | 10 | 2 | 24 |
| B43 | A52 | 7 | 2 | 24 | 8 | 2 | 24 |
| B44 | A53 | 5 | 2 | 24 | 8 | 2 | 24 |
| B45 | A54 | 6 | 2 | 24 | 7 | 2 | 24 |
| B46 | A58 | 7 | 2 | 24 | 9 | 2 | 24 |
| B47 | A59 | 8 | 2 | 24 | 9 | 2 | 24 |
| B48 | A55 | 6 | 2 | 24 | 7 | 2 | 24 |
| B49 | A57 | 8 | 2 | 24 | 8 | 2 | 24 |

Tabelle 5:

| Beispiel Nr. | Verbindung von Beispiel Nr. | Formulierung I | | | Formulierung II | | |
|---|---|---|---|---|---|---|---|
| | | LKS | Farbumschlag | Bildstabilität (h) | LKS | Farbumschlag | Bildstabilität (h) |
| B50 | A60 | 6 | 2 | 24 | 8 | 2 | 24 |
| B51 | A61 | 6 | 2 | 24 | 9 | 2 | 24 |
| B52 | A62 | 7 | 2 | 24 | 8 | 2 | 24 |
| B53 | A63 | 0 | 2 | 24 | 6 | 2 | 24 |
| B54 | A64 | 4 | 1 | 24 | 7 | 1 | 24 |
| B55 | A12 | 9 | 2 | 24 | 9 | 2 | 24 |
| B56 | A13 | 8 | 2 | 24 | 9 | 2 | 24 |
| B57 | A14 | 5 | 2 | 24 | 8 | 2 | 24 |
| B58 | A15 | 6 | 2 | 24 | 8 | 2 | 24 |
| B59 | A16 | 9 | 2 | 24 | 9 | 2 | 24 |
| B60 | A17 | 5 | 2 | 24 | 8 | 2 | 24 |
| B61 | A18 | 8 | 3 | 24 | 9 | 3 | 24 |
| B62 | A19 | 7 | 2 | 24 | 9 | 2 | 24 |
| B63 | A20 | 8 | 2 | 24 | 9 | 2 | 24 |

Tabelle 5:

| Beispiel Nr. | Verbindung von Beispiel Nr. | Formulierung I | | | Formulierung II | | |
|---|---|---|---|---|---|---|---|
| | | LKS | Farbumschlag | Bildstabilität (h) | LKS | Farbumschlag | Bildstabilität (h) |
| B64 | A21 | 5 | 2 | 24 | 8 | 2 | 24 |
| B65 | A22 | 8 | 2 | 24 | 9 | 2 | 24 |
| B66 | A23 | 9 | 2 | 24 | 9 | 2 | 24 |
| B67 | A24 | 7 | 2 | 24 | 9 | 2 | 24 |
| B68 | A25 | 5 | 2 | 24 | 8 | 2 | 24 |
| B69 | A26 | 8 | 3 | 24 | 8 | 3 | 24 |
| B70 | A27 | 4 | 2 | 24 | 7 | 2 | 24 |
| B71 | A85 | 8 | 3 | 24 | 9 | 3 | 24 |
| B72 | A29 | 9 | 3 | 24 | 10 | 3 | 24 |
| B73 | A97 | 6 | 2 | 24 | 8 | 2 | 24 |
| B74 | A30 | 9 | 3 | 24 | 10 | 3 | 24 |

**Ansprüche**

1. Verbindungen der Formel I

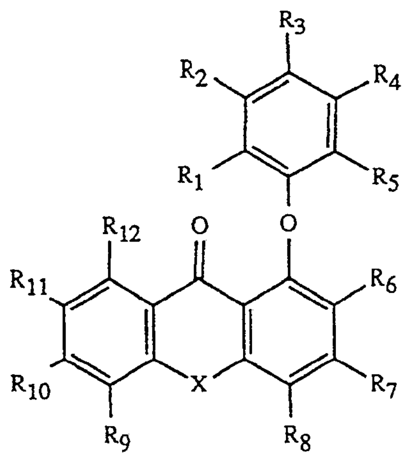

(I),

worin
X für O, S, SO, $SO_2$ oder $NR_{13}$ steht,
$R_1$ bis $R_5$ unabhängig voneinander für H, $C_1$-$C_{12}$-Alkyl, $C_1$-$C_{12}$-Alkoxy, $C_1$-$C_{12}$-Alkylthio, $C_1$-$C_4$-Aklyl-SO—, $C_1$-$C_4$-Alkyl-$SO_2$—, Halogen, $CF_3$, —CN, —$NO_2$, —OH, —$COOR_{14}$, —$CON(R_{15})_2$, oder—$N(R_{15})_2$ oder den Rest der Formel

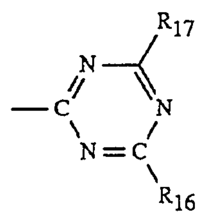

bedeuten, worin $R_{16}$ und $R_{17}$ unabhängig voneinander $C_1$-$C_6$-Alkyl, Phenyl, $C_1$-$C_6$-Alkylphenyl oder ($C_1$-$C_6$-Alkyl)$_2$-phenyl bedeuten, oder $R_3$ und $R_4$ oder $R_4$ und $R_5$ je zusammen —CH=CH-CH=CH— darstellen,
$R_6$ bis $R_{12}$ unabhängig voneinander H, $C_1$-$C_{12}$-Alkyl, $C_1$-$C_{12}$-Alkoxy, $C_1$-$C_{12}$-Alkylthio, $C_1$-$C_{12}$-Alkyl-SO—, $C_1$-$C_{12}$-Alkyl-$SO_2$—, in 1-Stellung mit insgesamt ein oder zwei —CN und/oder—$COOR_{14}$ substituiertes $C_1$-$C_4$-Alk-1-yl oder Benzyl, $C_6$-$C_{10}$-Aryl, $C_7$-$C_{14}$-Aralkyl, $C_6$-$C_{10}$-Aryl-CO, $C_6$-$C_{10}$-Aryloxy, $C_6$-$C_{10}$-Arylthio, $C_6$-$C_{10}$-Aryl-SO—, $C_6$-$C_{10}$-Aryl-$SO_2$, $C_7$-$C_{14}$-Aralkyloxy, $C_7$-$C_{14}$-Aralkylthio,, $C_7$-$C_{14}$-Aralkyl-SO—, $C_7$-$C_{14}$-Aralkyl-$SO_2$—, $C_1$-$C_{18}$-Acyl-O—, —$COOR_{14}$, —$CON(R_{15})_2$, $C_1$-$C_{18}$-Acyl-$NR_{15}$—, $(R_{15})_2N$—, Halogen, —$CF_3$ oder —CN oder je zwei benachbarte Reste von $R_6$ bis $R_{12}$ die Gruppen —CO-O-CO— oder —CO-$NR_{13}$-CO— bedeuten, wobei die Arylreste und Arylgruppen enthaltenden Reste unsubstituiert oder mit Halogen, —CN, —$CF_3$, $C_1$-$C_6$-Alkylthio, $C_1$-$C_6$-Alkoxy, $C_1$-$C_6$-Alkyl, —$COOR_{14}$, —CO-$N(R_{15})_2$ oder $C_1$-$C_{12}$-Acyl-O— substituiert sind,
$R_{13}$H, $C_1$-$C_{12}$-Alkyl oder $C_1$-$C_{12}$-Acyl, Phenyl oder Benzyl darstellt, die unsubstituiert oder mit $C_1$-$C_6$-Alkyl, $C_1$-$C_6$-Alkoxy, $C_1$-$C_6$-Alkylthio, Halogen oder —$COOR_{14}$ substituiert sind,
beide $R_{15}$ zusammen $C_4$-$C_8$-Alkylen, 3-Oxa-1,5-pentylen, 3-Thia-1,5-pentylen, 1,3-Butadien-1,4-diyl oder 2-Aza-1,3-Butadien-1,4-diyl bedeuten, oder die $R_{15}$ unabhängig voneinander H, $C_1$-$C_{12}$-Alkyl, oder unsubstituiertes oder mit $C_1$-$C_6$-Alkyl, $C_1$-$C_6$-Alkoxy, $C_1$-$C_6$-Alkylthio, Halogen oder—$COOR_{14}$ substituiertes Phenyl oder Benzyl darstellen, und $R_{14}$ H oder der um die OH-Gruppe verminderte Rest eines aromatischen oder aliphatischen Alkohols ist,
mit Ausnahme von 1-Phenoxy-4-methylxanthen-9-on.

2. Verbindungen gemäss Anspruch 1, worin in Formel I $R_1$ bis $R_5$ unabhängig voneinander H, $C_1$-$C_6$-Alkyl, $C_1$-$C_6$-Alkoxy, Halogen, —$CF_3$, —ON, —$NO_2$, —$COOR_{14}$, —OH, —$N(R_{15})_2$, oder $R_3$ und $R_4$ oder $R_4$ und

$R_5$ je zusammen —CH=CH-CH=CH— bedeuten, $R_{15}$ je für $C_1$-$C_6$-Alkyl steht und $R_{14}$ lineares oder verzweigtes $C_1$-$C_{18}$-Alkyl darstellt.

3. Verbindungen gemäss Anspruch 2, worin in Formel I mindestens 2 der Reste $R_1$ bis $R_5$ für H stehen.

4. Verbindungen gemäss Anspruch 1, worin in Formel I $R_6$ bis $R_{12}$ unabhängig voneinander H, $C_1$-$C_6$-Alkyl, $C_1$-$C_6$-Alkoxy, $C_1$-$C_6$-Alkylthio, $C_1$-$C_6$-Alkyl-SO—, $C_1$-$C_6$-Alkyl-$SO_2$, in 1-Stellung mit insgesamt ein oder 2 —CN und/oder —$COOR_{14}$ substituiertes $C_1$— oder $C_2$-Alkyl, Phenyl, Phenyl-$C_1$-$C_4$-Alkylen, Phenyl-CO—, Phenyloxy, Phenylthio, Phenyl-SO—, Phenyl-$SO_2$—, Benzyloxy, Benzylthio, Benzyl-SO—, Benzyl-$SO_2$—, $C_1$-$C_8$-Acyl-$NR_{15}$—, —$COOR_{14}$, —$CON(R_{15})_2$, $(R_{15})_2N$—, —F, —Cl, —Br, —$CF_3$ oder —CN oder je zwei benachbarte Reste von $R_6$ bis $R_{12}$ zusammen —CO-O-CO— oder —CO-$NR_{13}$-CO— bedeuten, $R_{13}$ $C_1$-$C_6$-Alkyl bedeutet, oder Phenyl oder Benzyl darstellen, die unsubstituiert oder mit $C_1$-$C_4$-Alkyl, $C_1$-$C_4$-Alkoxy, F, Cl, Br oder —$COOR_{14}$ substituiert sind,

$R_{14}$ H, lineares oder verzweigtes $C_1$-$C_{18}$-Alkyl, Phenyl, Benzyl oder $-(C_nH_{2n}-O)_m-R_{18}$ ist, wobei n eine ganze Zahl von 2 bis 6 und m eine Zahl von 1 bis 20 darstellen, und $R_{18}$ H, $C_1$-$C_{18}$-Alkyl, Cyclohexyl, Phenyl oder Benzyl bedeutet, und $R_{15}$ $C_1$-$C_6$-Alkyl bedeutet, oder Phenyl oder Benzyl ist, die unsubstituiert oder mit $C_1$-$C_4$-Alkyl, $C_1$-$C_4$-Alkoxy, —F, —Cl, —Br oder —$COOR_{14}$ substituiert sind, oder beide $R_{15}$ zusammen 1,4-Butylen, 1,5-Pentylen, 3-Oxa-1,5-pentylen oder 1,3-Butadien-1,4-diyl darstellen.

5. Verbindungen gemäss Anspruch 1, worin in Formel I mindestens zwei der Reste $R_6$ bis $R_{12}$ für H stehen.

6. Verbindungen gemäss Anspruch 1, worin in Formel I $R_6$ für H, —Cl, —Br oder Methyl steht.

7. Verbindungen gemäss Anspruch 1, worin in Formel I $R_9$ H oder —Cl bedeutet.

8. Verbindungen gemäss Anspruch 1, worin in Formel I $R_{11}$ H oder —Cl bedeutet.

9. Verbindungen gemäss Anspruch 1, worin in Formel I $R_{10}$ und/oder $R_{12}$ —Cl, —Br, unsubstituiertes oder substituiertes Phenyloxy oder —COO-($C_1$-$C_{12}$-Alkyl) bedeuten.

10. Verbindungen gemäss Anspruch 1, worin in Formel I $R_6$ für H, —Cl, —Br oder Methyl steht, oder worin in Formel I $R_9$ H oder —Cl bedeutet, oder worin in Formel I $R_{11}$ H oder —Cl bedeutet, oder worin in Formel I $R_{10}$ und/oder $R_{12}$ —Cl, —Br, unsubstituiertes oder substituiertes Phenyloxy oder —COO-($C_1$ -$C_{12}$-Alkyl) bedeuten, oder worin in Formel I $R_7$ und/oder $R_8$ für H, —F, —Cl, —$NO_2$, —$CF_3$, —Br, $C_1$-$C_4$-Alkyl, $C_1$-$C_4$-Alkoxy, Phenyl-CO—, α-Cyanobenzyl, $C_1$-$C_8$-Acyl-$NR_{15}$—, Pyrryl, —COO-($C_1$-$C_{12}$-Alkyl) stehen, oder Phenoxy, Phenylthio oder Phenylsulfonyl bedeuten, die unsubstituiert oder mit —F, —Cl, $C_1$-$C_4$-Alkyl, $C_1$-$C_4$-Alkoxy, —COOH oder —COO-($C_1$-$C_{12}$-Alkyl) substituiert sind, oder $R_7$ und $R_8$ zusammen

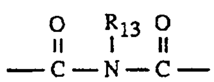

darstellen, und $R_{13}$ H oder $C_1$-$C_6$-Alkyl bedeutet.

11. Verbindungen gemäss Anspruch 1, worin in Formel I $R_{13}$ $C_1$-$C_4$-Alkyl oder $C_1$-$C_8$-Acyl darstellt, oder Phenyl oder Benzyl ist, die unsubstituiert oder mit F oder —$COOR_{14}$ substituiert sind, und $R_{14}$ für H oder $C_1$-$C_{12}$-Alkyl steht.

12. Verbindungen gemäss Anspruch 1, worin in Formel I $R_{14}$ H, $C_1$-$C_{18}$-Alkyl, $-(C_nH_{2n}-O)_m-H$, Phenyl, $C_1$-$C_6$-Alkylphenyl, Benzyl, $C_1$-$C_6$-Alkylbenzyl oder Cyclohexyl bedeutet, n eine ganze Zahl von 1 bis 6 und m eine Zahl von 1 bis 20 darstellen.

13. Verbindungen gemäss Anspruch 1, worin in Formel I $R_{15}$ H, $C_1$-$C_6$-Alkyl, Phenyl, Benzyl, oder beide $R_{15}$ zusammen Tetramethylen, Pentamethylen, 3-Oxa-1,5-pentylen oder 1,3-Butadien-1,4-diyl bedeuten.

14. Verbindungen gemäss Anspruch 1, worin $R_1$ bis $R_5$ H oder $R_1$, $R_2$, $R_4$ und $R_5$ H und $R_3$ —$CO_2C_2H_5$ bedeuten,

$R_6$ und $R_8$ Cl und $R_7$ und $R_9$-$R_{12}$ H darstellen, und X für $NCH_3$ oder N-n-$C_3H_7$ steht.

**15.** Verfahren zur Herstellung von Verbindungen der Formel I gemäss Anspruch 1, dadurch gekennzeichnet, dass man eine Verbindung der Formel II

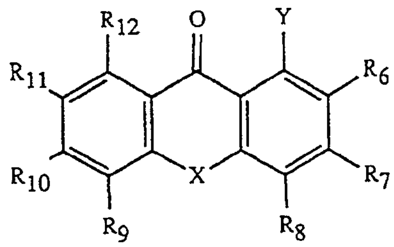

(II),

worin X und $R_6$ bis $R_{12}$ die in Anspruch 1 angegebenen Bedeutungen haben und Y —$NO_2$ oder Halogen darstellt, in Gegenwart eines dipolaren aprotischen oder protischen Lösungsmittels bei erhöhter Temperatur mit einer Verbindung der Formel III

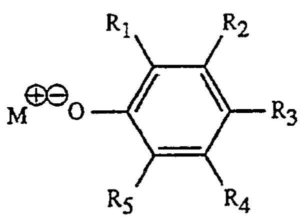

(III)

umsetzt, worin $R_1$ bis $R_5$ die in Anspruch 1 angegebenen Bedeutungen haben und $M^{\oplus}$ für ein Alkalimetallkation, ein quaternäres Ammonium— oder ein Phosphoniumkation steht.

**16.** Strahlungsempfindliche Zusammensetzung, enthaltend
a) ein strahlungsempfindliches organisches Material, und
b) mindestens eine Verbindung der Formel I gemäss Anspruch 1, einschliesslich 1-Phenoxy-4-methylxanthen-9-on.

**17.** Zusammensetzung gemäss Anspruch 16, dadurch gekennzeichnet, dass die Verbindung der Formel I in einer Menge von 0,001 bis 20 Gew.-% enthalten ist, bezogen auf die Komponente a).

**18.** Zusammensetzung gemäss Anspruch 16, dadurch gekennzeichnet, dass es sich bei dem strahlungempfindlichen organischen Material um a1) eine nichtflüchtige monomere, oligomere oder polymere Substanz mit photopolymerisierbaren oder photodimerisierbaren ethylenisch ungesättigten Gruppen, a2) um ein kationisch härtbares System oder a3) um photovernetzbare Polyimide handelt.

**19.** Zusammensetzung gemäss Anspruch 18, dadurch gekennzeichnet, dass Komponente a1) ein Homo— oder Copolymer von Acryl—, Methacryl— oder Maleinsäureestern ist, deren Estergruppen einen Rest der Formel

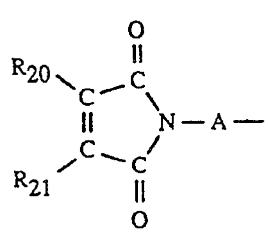

enthalten, worin A für unsubstituiertes oder mit Hydroxyl substituiertes lineares oder verzweigtes $C_2$-$C_{12}$-Alkylen, Cyclohexylen oder Phenylen steht, und $R_{20}$ und $R_{21}$ unabhängig voneinander Cl, Br, Phenyl oder $C_1$-$C_4$-Alkyl bedeuten, oder $R_{20}$ und $R_{21}$ zusammen Trimethylen, Tetramethylen oder

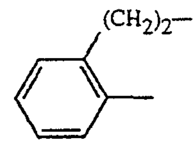

bedeuten.

**20.** Zusammensetzung gemäss Anspruch 18, dadurch gekennzeichnet, dass Komponente a2) eine Epoxidverbindung mit mindestens 2 Epoxidgruppen im Molekül ist, der ein Photoinitiator einverleibt ist.

**21.** Zusammensetzung, enthaltend
a) ein farbloses organisches Lösungsmittel, ein Polymer oder ein organisches Glas oder ein Verbundglas, und
b) eine Verbindung der Formel I gemäss Anspruch 1, einschliesslich 1-Phenoxy-4-methyl-xanthen-9-on.

**22.** Verfahren zur Herstellung von gefärbten Materialien unter Einwirkung von Licht, dadurch gekennzeichnet, dass man dem Material eine Verbindung der Formel I gemäss Anspruch 1 einschliesslich 1-Phenoxy-4-methylxanthen-9-on einverleibt und darauf das Material mit Licht bestrahlt.

**23.** Verwendung von Verbindungen der Formel I gemäss Anspruch 1 einschliesslich 1-Phenoxy-4-methylxanthen-9-on als Photosensibilisatoren und Farbindikatoren oder als Photoschaltelemente bei Lichteinwirkung.